# EUROPEAN PATENT SPECIFICATION

(11) **EP 3 244 758 B1**
(45) Date of publication and mention of the grant of the patent: **19.08.2020**
(21) Application number: 16737117.8
(22) Date of filing: 12.01.2016
(51) Int. Cl.: A23L 5/20, A23L 7/104, A21D 8/04, A21D 13/00, A23L 33/10, C12P 39/00, A23L 7/00, A61K 35/744, A61K 35/747, A21D 10/00, C12P 1/00, A23L 33/125

(54) **LOW-FRUCTAN GRAIN MATERIAL AND A METHOD FOR PRODUCING THE SAME**
GETREIDE MIT NIEDRIGEM FRUCTANGEHALT UND VERFAHREN ZUR HERSTELLUNG DAVON
MATÉRIAU À BASE DE GRAINS À FAIBLE TENEUR EN FRUCTANE ET PROCÉDÉ POUR LE PRODUIRE

(30) Priority: 12.01.2015 FI 20155018
(43) Date of publication of application: 22.11.2017
(73) Proprietor: OY Karl Fazer AB, 00941 Helsinki (FI)
(72) Inventor: LOPONEN, Jussi, 01450 Vantaa (FI)
(74) Representative: Laine IP Oy
(86) International application number: PCT/FI2016/050011
(87) International publication number: WO 2016/113465

(56) References cited:
- EP-A1- 1 258 526
- EP-A1- 2 103 219
- EP-A2- 1 084 624
- WO-A1-2014/179843
- JP-A- 2006 149 228
- FRETZDORFF B ET AL: "Reduction of endogenous fructans during rye whole-meal breadmaking", GETREIDE MEHL UND, BOCHUM, DE, vol. 57, no. 3, 1 January 2003 (2003-01-01), pages 147-151, XP009111230, ISSN: 0367-4177
- J. R. BIESIEKIERSKI ET AL: "Quantification of fructans, galacto-oligosacharides and other short-chain carbohydrates in processed grains and cereals : Short-chain carbohydrates in grains and cereals", JOURNAL OF HUMAN NUTRITION AND DIETETICS, vol. 24, no. 2, 21 February 2011 (2011-02-21), pages 154-176, XP055480959, GB ISSN: 0952-3871, DOI: 10.1111/j.1365-277X.2010.01139.x
- ANONYMOUS.: 'Using sourdough breads to reduce fructans.' 01 February 2014, XP055467202 Retrieved from the Internet: <URL:http://cooking.stackexchange.com/quest ions/41262/using-sourdough- breads-to-reduce-fructans>
- MÜLLER, M. ET AL.: 'Fermentation of fructans by epiphytic lactic acid bacteria.' JOURNAL OF APPLIED BACTERIOLOGY vol. 76, no. 4, 1994, pages 406 - 411, XP000982620
- VAN DER MEULEN, R. ET AL.: 'Population dynamics and metabolite target analysis of lactic acid bacteria during laboratory fermentations of wheat and spelt sourdoughs.' APPLIED AND ENVIRONMENTAL MICROBIOLOGY vol. 73, no. 15, 2007, pages 4741 - 4750, XP055467205
- JASINSKA-KULIGOWSKA, I. ET AL.: 'Effect of fermentation, extrusion and baking processes on content of fructans in rye products.' ZYMNOSC. vol. 5, 2013, pages 129 - 141, XP055467206
- ANDERSSON, R. ET AL.: 'Content and molecular-weight distribution of dietary fiber components in whole-grain rye flour and bread.' JOURNAL OF AGRICULTURAL AND FOOD CHEMISTRY vol. 57, no. 5, 2009, pages 2004 - 2008, XP055467207
- WHELAN, K. ET AL.: 'Fructan content of commonly consumed wheat, rye and gluten-free breads.' INTERNATIONAL JOURNAL OF FOOD SCIENCES AND NUTRITION vol. 62, no. 5, 2011, pages 498 - 503, XP055467210

## Description

### FIELD OF THE INVENTION

The present invention is directed to a technology that allows efficient removal of fructan from grain raw material. The method according to the invention utilizes a seed starter or a microbial starter to decrease the fructan content. Said seed starter or microbial starter is generated from grain material having a low content of damaged starch. The grain material obtained by the method of the invention has fructan content significantly lower compared to the starting situation. This low-fructan grain material can be used in producing low-fructan grain ingredients, products suitable e.g. for low-FODMAP diet, and various cereal food products with dietary benefits.

### BACKGROUND OF THE INVENTION

Digestion-related problems are a frequent cause of general and social discomfort. These problems cover a diverse selection of gastrointestinal symptoms of which bloating, gas production, abdominal pain, overall discomfort, constipation, and loose stools are among the most frequent. Today many of the sufferers of such symptoms are believed to suffer from irritable bowel syndrome (IBS). IBS is clearly more frequent in women and it is believed to concern 10 - 20 % of Western population; i.e. IBS is more frequent in Western population than lactose-intolerance (many people having lactose intolerance, though, might have IBS and vice versa).

Currently there is no good medical cure for IBS. Much attention has been paid on dietary management of IBS. Most attention has been laid on a diet called LOW-FODMAP diet. The idea of the diet is to avoid food items that contain FODMAP compounds. Term FODMAP is derived from "Fermentable, Oligo-, Di-, Monosaccharides, and Polyols". FODMAPs are short chain carbohydrates and monosaccharides which are poorly absorbed in the small intestine. FODMAP compounds include fructans, galactans, fructose, and polyols.

Common sources of fructans include for example wheat, rye, onion, and garlic. Some examples of fructan contents of grains are as follows: rye (bran) 7 % (on grain material basis), rye (grain) 3 - 7 %, and wheat flour 1 - 4 %. Although wheat is not generally considered as being especially rich in FODMAP compounds, its relatively high consumption rate makes it a relevant source of fructans. This is why the FODMAP diet guidelines instruct to avoid wheat. Rye consumption is high in Northern Europe. Rye bread contains more FODMAP compounds compared to wheat bread, because whole grain rye contains more fructans than wheat flour.

Fructans are built up of fructose residues, normally with a terminal sucrose unit (i.e. a glucose-fructose disaccharide). The linkage position of the fructose residues determines the type of the fructan. The basic types of single-linkage fructans are inulin and levan (or phlein). Additionally, there exists mixed-linkage fructan called graminan.

Some prior art related to levels of fructan in bread is existing. In the article by Andersson *et al.* (2009) it was shown that the yeast fermented bread and especially the sourdough bread had lower contents of fructan as compared to whole grain rye flour. The results of Andersson *et al.* show that the fructan content of whole grain rye can be reduced from 5.0 % to 1.9 % by sourdough (62% reduction) and to 3.4 % by yeast fermentation (32% reduction). The results also show that fructans are degraded during the bread-making process resulting in lower contents of total and extractable dietary fiber in the bread.

Article by Rakha *et al.* (2010) discloses that during bread making, the low-molecular weight fraction of fructan is most available for degradation by yeast or by endogenous enzymes present in the ingredients. According to Rakha *et al.* the fructan content in rye milling fractions ranges from 3.4 % in inner endosperm to 5.0 % in bran. The fructan content of rye breads varied from 1.9 % to 4.0 %, with an average of 2.8 % in crisp breads, with a sample containing only whole grain rye flour being the highest in fructan content.

The dough according to US patent application US 2011/0129572 A1 comprises at least one fructose-containing polysaccharide and at least one enzyme capable of degrading said polysaccharide into short-chained fructo-oligosaccharide and fructose. The baked product produced using this dough was said to have an increased softness compared to otherwise identical control bread or baked product produced using dough not containing the enzyme. Patent publication EP 2103219 A1 discloses a dough comprising rye flour and at least one enzyme capable of degrading fructose-containing polysaccharides into short-chained fructo-oligosaccharides and fructose. Article by Fretzdorff *et al* (2003) discloses sourdoughs having a fructan content, which at its lowest is 2.01%. Patent publication WO 2014/179843 A1 focuses on gluten enrichment of low fructan flours for irritable bowel syndrome sufferers.

The discovery related to lowering the fructan amounts in plant material of patent application EP 1084624 is that while *Lactobacillus* strains in general do not degrade fructan, there are *Lactobacillus* strains which do have this property. According to EP 1084624 those strains are preferably *Lactobacillus paracasei* and *Lactobacillus plantarum.*

As can be noted from above, some techniques to alter fructan levels are currently known and used. Additionally, it is known that sour bread has naturally lower levels of fructan. These fructan lowering techniques are generally based on using fermentation or specific enzymes. However, with the use of fructan-degrading enzymes there is a possibility that fructo-oligosaccharides (FOS) are formed as degradation products as by this mean not all fructan is converted to fructose.

FOS are carbohydrates that the human body cannot fully digest and can thus function as prebiotics. There are some positive effects suggested for FOS. They may for example produce substances that stop the growth of harmful, toxic gram-negative and positive bacteria in the intestines. However, according to the currently available scientific evidence FOS can execute some harmful effects. FOS can cause e.g. bloating, flatulence, abdominal and intestinal discomfort, and eructation. Furthermore, people with lactose intolerance were shown to particularly suffer from these side effects. The reason for these symptoms may be that FOS are generally gastrointestinally more active than fructan polymer, since the intestinal microflora ferments them more rapidly. Moreover, fructose is also considered being a FODMAP-compound, especially when no comparable amount of glucose is present in the food item or meal. This is because fructose absorption in human body occurs along with glucose-induced uptake system. If fructose is not absorbed it will be fermented by intestinal microflora. Furthermore, mannitol (a polyol) formation typically takes place in fermentation in the presence of heterofermentative lactobacilli and a fructose source. This is because heterofermentative lactobacilli use fructose as an electron acceptor and, thus, convert it to mannitol. This practically means that a relevant amount of mannitol is present in many fermented grain products.

What are still needed in the art are grain materials that are substantially free of fructan and thereby can be used to prepare products that are suitable for low-FODMAP diets. What is also still needed in the art is an efficient method for fructan removal from grain material that would not result in unfavorable degradation products and further metabolites, such like FOS, fructose, or mannitol in the end product. Therefore, a method that would enable the efficient removal of fructan without unfavorable degradation products and metabolites would be very beneficial for development of food products suitable for low-FODMAP diet. Consumption of these food products would not cause gastrointestinal problems. Said food products could even have a positive effect on gastrointestinal health and in that way to general wellbeing.

### SUMMARY OF THE INVENTION

The invention is defined by the features of the independent claims. Some specific embodiments are defined in the dependent claims.

According to a first aspect of the present invention, there is provided a method for producing low-fructan grain material by means of lactic acid bacteria, said method comprising the steps of
- generating a seed starter from grain material by back slopping at least 6 times, wherein said grain material has a damaged starch content less than 0.5% by weight of said grain material, wherein the damaged starch content refers to a proportion of broken starch granules, to obtain the seed starter, which comprises lactic acid bacteria; and either
   1)- mixing grain raw material with liquid and with said seed starter;
      - incubating the obtained mixture at 20 to 50°C for 4 to 72 hours; and
      - recovering the low-fructan grain material, which comprises 0 to 0.5% by weight of fructan; or
   2)- isolating pure culture isolates from said seed starter, wherein the pure culture isolates comprise lactobacilli selected from *Lactobacillus ultunensis, L. crispatus, L. amylovorus, L. amylovorans, L. sobrius* and *L. acidophilus;*
      - mixing grain raw material with liquid and with said pure culture isolate(s);
      - incubating the obtained mixture at 20 to 50°C for 4 to 72 hours; and
      - recovering the low-fructan grain material, which comprises 0 to 0.5% by weight of fructan,
wherein said grain raw material is wheat, rye, or barley, or a mixture containing any of these.

The present invention is directed to a technology that allows efficient removal of fructan from grain material. The raw material of the invention is grain material from wheat, barley or rye or mixtures containing any of these. In the method according to the present invention the grain raw material is soaked into liquid and pure culture isolate(s) (a microbial starter) or a seed starter is added to the mixture. The obtained mixture is then allowed to incubate.

In a further aspect of the present invention, there is provided low-fructan grain material obtainable by the methods according to the invention. Thus the present invention is also directed to wheat, rye and barley grain materials that are obtainable by the methods according to the invention and comprise 0 to 0.5% by weight of fructan. More specifically the present invention is directed to low-fructan grain material comprising 0 to 0.5% by weight of fructan. The low-fructan grain material of the present invention can be used to prepare products that are suitable for low-FODMAP diet products.

As stated above, the method for producing low-fructan grain material according to alternative 1) of the present invention comprises the steps of mixing the grain raw material with liquid and with a seed starter generated from grain material having a damaged starch content less than 0.5% by weight of said grain material by back slopping, and incubating the obtained mixture. A seed starter or a microbial starter used in the method according to the invention comprises lactic acid bacteria and has the ability to consume more than 90%, preferably more than 95% of fructan from the raw grain material when the mixture is incubated at 20 to 50°C for 4 to 72 hours. After the incubation, the soaked grain material has fructan content significantly lower compared to that of the grain raw material.

Further processing of the grain material is usually carried out and can include for instance wet-milling, pasteurization, extrusion, drying or dry-milling, or acidity adjustment. The obtained low-fructan grain material can be used for producing various low-fructan products, and especially those that are suitable for low-FODMAP diet, for instance baked goods. Use of the low-fructan grain material obtainable by the methods of the present invention for making dough is therefore also an aspect of the present invention.

### BRIEF DESCRIPTION OF THE FIGURES

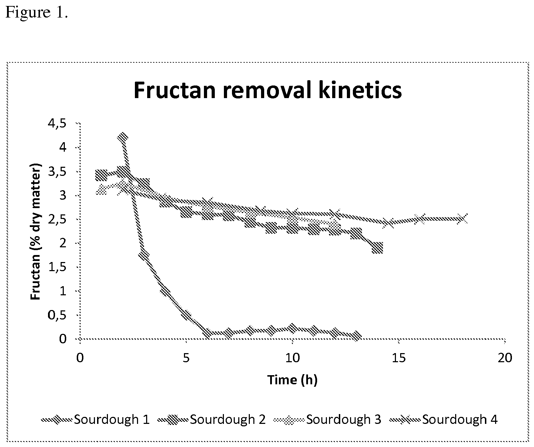

Figure 1. The decrease in fructan content of whole rye flour by using different seed starters is presented as a function of time. The seed starter of sourdough-1 was prepared using rye material virtually free from damaged starch and fermenting temperature of 45°C. The other three seed starters were prepared using rye material with damaged starch. These were fermented at 30°C (sourdough-2, sourdough-3) and at 45°C (sourdough-4).

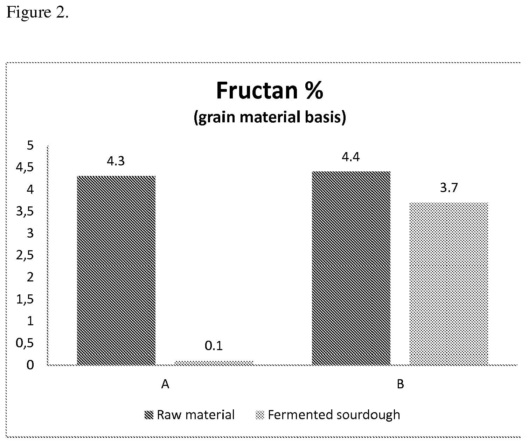

Figure 2. Fructan contents of the sourdoughs prepared using cereal material virtually free from damaged starch (A) vs. similar sourdough system prepared using cereal material containing damaged starch (B) were shown to be 0.1 % and 3.7 % (on grain material basis) compared to fructan contents of 4.3 % and 4.4 % of the raw grain material, respectively.

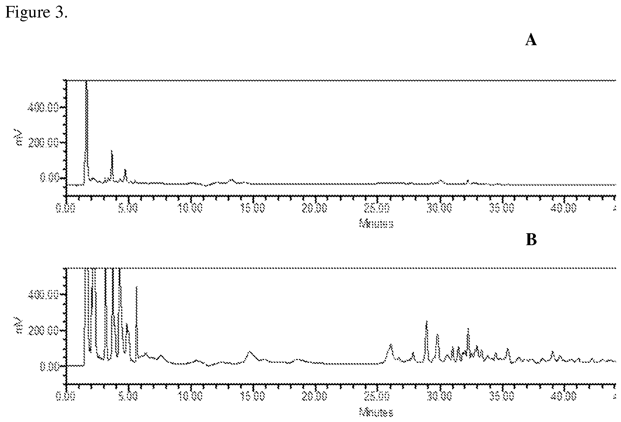

Figure 3. Oligosaccharide profiles of sourdough prepared using cereal material virtually free from damaged starch (A) vs. similar sourdough system prepared using cereal material containing damaged starch (B). Peaks between 25 and 40 min represent mostly FOS.

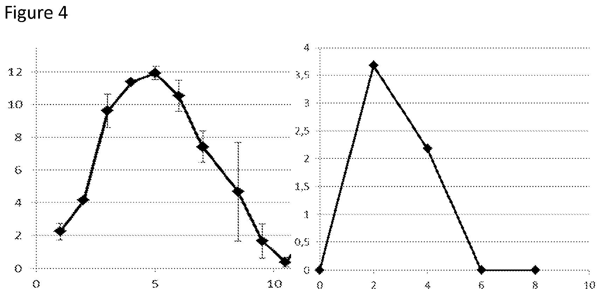

Figure 4. Development of mannitol and fructose concentrations during the incubation of cut rye kernels and water with fructan degrading seed starter. Left-side panel: Mannitol concentrations in g/kg. Right-side panel: Fructose concentration in % of dry matter.

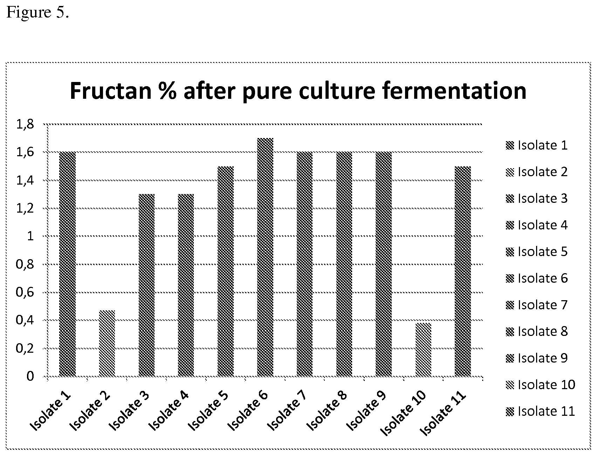

Figure 5.11 microbe colonies were isolated from seed starter that was capable to efficiently reduce fructan levels. The microbes of these colonies were analyzed and their capability to consume fructan was measured.

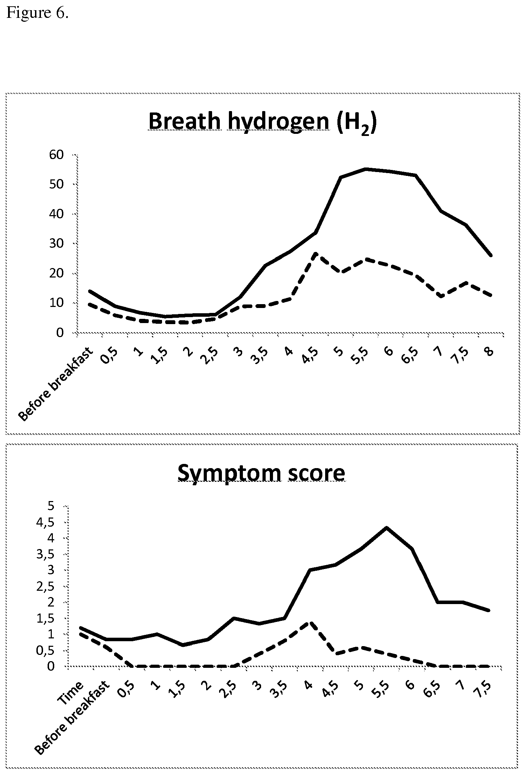

Figure 6. Solid lines represent normal rye pumpernickel bread with fructan content of 2% and the dashed lines represent rye pumpernickel bread with reduced fructan content of 0.5%. The upper panel presents breath hydrogen levels after the breakfast and the lower panel presents the cumulative symptom scores as a function of time after breakfast

### DETAILED DESCRIPTION OF THE INVENTION

This invention comprises efficient methods for fructan removal from grain material. Said methods do not result in an unfavorable degradation of fructan resulting in unfavorable degradation products and metabolites, such as FOS, fructose and mannitol.

This invention provides wheat, rye and barley grain materials that comprise 0 to 0.5% by weight of fructan and thereby can be used to prepare products that are suitable for a specific diet such as low-FODMAP diet. The present invention provides low-fructan wheat, rye or barley grain material comprising 0 to 0.5 % by weight of fructan. Preferably, said grain material comprises 0 to 0.3 % by weight of fructan. More preferably said grain material comprises 0 to 0.1 % by weight of fructan.

According to the present invention, an efficient removal of fructan from grain raw material is achieved. The grain raw material is wheat, barley or rye, or a mixture containing any of these. Closely relative grains to wheat, barley and rye, such as spelt, emmer, kamut, tritica-le etc. or mixtures containing any of these can also be used. In this description, a "kernel" means the seed of the cereal plant, while "grain" means cereal material in general. Whole grain products are made from the whole kernel.

The grain raw material according to the present invention can comprise rye, wheat or barley in the form of meal, bran, kernels, cut kernels, polished kernels, crushed grain, semolina, or bulgur. Oat as a raw material is not included within the scope of this invention. However, oat can be included in flour mixes used in baking processes utilizing low-fructan grain material obtained by the method of the present invention. The grain raw material used in the method of the invention preferably contains at least 1 % (w/w) fructan. However, also grain raw material with lower fructan content can be included in flour mixes used in food processes, such as baking or extrusion that utilize low-fructan grains obtained by the method of the present invention.

### Preparation of the seed starter

The seed starter with the ability to consume fructan quantitatively can be produced by utilizing back slopping. Back slopping means that small quantities of dough from the manufacture of a fermented product from a previous batch are used as the inoculum or starter for the subsequent batch production. Grain material can be added to liquid, preferably water, in a ratio that can be 1 : 0.6 or 1 : 10 or anything between these. The ratio is preferably 1 : 1.5 when the grain material is rye. The ratio is preferably 1 : 1 when the grain material is wheat.

In a typical flour milling process, a proportion of starch granules gets broken down, i.e. damaged starch is formed. For instance, a typical wheat flour milling process can result in damaged starch content of 5% or more. In the present invention, in the preparation of the seed starter it is essential that the grain material has a low content of damaged starch, *i.e.* the damaged starch content of the grain material is less than 0.5% by weight of said grain material. Preferably, the damaged starch content of the grain material is less than 0.25%, or even less than 0.1% by weight of said grain material.

The liquids in which the raw grain material can be soaked include water, carbonated water, milk, sour milk, cultured buttermilk, milk kefir, coconut kefir, water kefir, cultured yogurt, whey, lemon juice, apple cider vinegar, dilute acid or base, or buffer. A preferred liquid is water.

The mixture is incubated at 20 - 50°C for 4 to 72 hours. Preferably, the incubation temperature is 40 - 45°C and time is 6 to 16 hours. Next day, a fresh batch of the grain material and liquid, preferably water, is mixed as above and inoculated with 1 - 10% of the previously incubated mixture. The incubation time and temperature are the same as in the previous step. This back slopping is done at least 6 times and can be continued as long as necessary or wanted.

The outcome of backslopping started from grain material having a low content of damaged starch is the formation of spontaneous microflora that is able to efficiently utilize fructans as a carbohydrate and quantitatively to consume (and thereby remove) fructans from grain raw material.

As described above, the seed starter can be produced spontaneously by continuous back slopping. It is important that the grain materials used for seed starter production have low content of damaged starch (e.g. whole kernels, peeled kernels, cut kernels). Damaged starch is starch formed during milling of kernel to flour as the mechanical stress induced by the milling process partially breaks down (damages) intact starch granules. The damaged starch formed is available for enzymatic hydrolysis by amylases, whereas undamaged starch is far more resistant to enzymatic hydrolysis. Enzymatic hydrolysis of damaged starch results in degradation products of starch, mostly maltose. In sourdoughs, there is amylase activity originated from grain material that is active against damaged starch. In addition, amylolytic microbes can be included in the sourdough microflora. In most sourdough systems that contain plenty of damaged starch, the maltose released by amylases is the main available nutritious carbohydrate. In the system where there is no damaged starch available to be enzymatically degraded to maltose that could serve as a nutrient, fructan is a major nutritious carbohydrate that is readily available. Therefore using grain materials with low amounts of damaged starch, for example below 0.5 % (on grain basis) of damaged starch, as a raw material in continuous back-slopping can result in microbial flora that is able to efficiently utilize fructan as a nutritious carbohydrate. This means that the adapted microflora has the ability to hydrolyze fructan and further use the possible degradation products and metabolites (fructose, FOS, mannitol) for growth. This means that the adapted microflora produces sourdough with low fructan content. The flora may also have transport system for fructans or their hydrolysis products.

### Preparation of low-fructan grain material

Accordingly, the present invention provides a method for producing low-fructan grain material, where in alternative 1) said method comprises the steps of mixing the grain raw material with liquid and with a seed starter obtained by back slopping from grains having a damaged starch content less than 0.5% by weight of said grains. Preferably, the damaged starch content of said grain raw material is less than 1.0%, more preferably less than 0.5%, even more preferably less than 0.25% or 0.1%.

The liquids in which the grain raw material can be soaked are the same as described above for the preparation of the seed starter. A preferred liquid is water. Incubation times and temperature used in the preparation of the low-fructan grain material according to the invention are the same as described above for the preparation of the seed starter.

In alternative 2), pure culture isolates (within this disclosure also "microbial starters"), derived from the seed starter generated from grain material having a low content of damaged starch by back slopping, can be used in the method according to the invention. Said seed starter-derived pure culture isolates are obtained from the seed starter by isolating bacterial colonies with different morphology to make pure cultures. When said microbial starter is used in the method according to the invention, the grain raw material may contain a higher damaged starch content than 1.0%.

In one embodiment of the invention, the pure culture isolates derived from the seed starter generated from grain material having a low content of damaged by back slopping can be used together with any other starter. These starters can be, for instance, commercial starters or pure culture starters, or seed starters derived from sourdough or other fermentations, such as fermented dairy or plant products.

Said seed starter or microbial starter has typically ability to consume or degrade (hydrolyze) more than 90% of fructan from the grain raw material when the mixture is incubated at 20 to 50°C for 4 to 72 hours. Preferably, said mixture is incubated at 40 - 45°C for 6 - 24 hours. Preferably after the incubation the soaked grain material has fructan content which is >90% lower compared to the fructan content of the grain raw material.

The microbial starter or the adapted microflora of the seed starter generated as described above comprises lactobacilli. Preferably, the seed starter or the microbial starter comprises or is *Lactobacillus ultunensis, L. crispatus, L. amylovorus, L. amylovorans, L. sobrius* and/or *L. acidophilus.*

The grain material obtained by fermentation/incubation is for example low-fructan rye, wheat or barley flour/meal/flakes/kernels, low-fructan cut grains, or low-fructan crushed grain with 0.5 %, 0.4 %, 0.3 %, 0.2 %, 0.1 %, or 0 % (w/w) of fructan. The obtained grain material contains 0 % to 0.5 % (w/w) of fructan, preferably 0 % to 0.3 % (w/w), and more preferably 0 % to 0.1 % (w/w) of fructan.

The obtained low-fructan grain material is substantially free also from FOS, fructose and mannitol. Within this disclosure, the expressions "substantially free from FOS, fructose and mannitol" and "substantially fructan-free" mean that the material does not contain said substances, or contains said substances at only very minor amounts (see e.g. Examples 5 and 6 and their results as shown in Figures 3 and 4). The amounts of FOS, fructose and mannitol in the low-fructan grain material according to the invention are at most 0.2% each.

The obtained low-fructan material may be further processed for instance by dry-milling, wet-milling, extrusion, drying or, pasteurizing, or its acidity level can be adjusted. The drying process can be for instance spray drying, drum drying, vacuum drying, extrusion drying, oven drying or freeze-drying. More preferably, the obtained low-fructan grain material is further dried. The obtained grain material can be used directly in baking process to produce baked goods with reduced fructan content. The present invention provides also the use of the obtained low-fructan grain material in making dough and in baking.

The LOW-FODMAP-DIET is becoming a dietary trend, which will be recognized by the consumers, especially those who feel they suffer from gastrointestinal problems or those having been diagnosed with IBS. The present invention makes it possible to produce a raw material for grain-based products (e.g. flour, bread, porridge, breakfast cereals or extruded snacks) that are substantially lower in their FODMAP content compared to their traditional counterparts. The product according to the invention is low-fructan wheat, rye, or barley material, i.e. flour, meal, crushed grain, kernels, cut kernels, flakes for bread, biscuits or porridge or extrudates or flour mixtures to be used industrially or at home. All kinds of baked products containing low-fructan grains can thus be produced using low-fructan grain material according to the invention. Specific examples of the products containing low-fructan grains according to the invention are bread, that can also be in the form of loaves or rolls, French baguette-type bread, pita bread, tortillas, cakes, pancakes, biscuits, cookies, pie crusts, crisp bread, steamed bread, pizza and the like. Low-fructan grains containing products also include biscuit, cracker crisp bread, flour, meal, flake, flour mix or blend, extrudate, breakfast cereal, pet food, cereal bar, porridge, energy bar, snack bar, crisp bar, dried sourdough powder, pasta, noodle, spaezle, bulgur, semolina, or couscous. More specifically, the product can be any of the above-mentioned products with 30 - 100% lower fructan content, preferably with 50-100% lower fructan content, compared to conventional corresponding products. Accordingly, the baked product may be substantially fructan-free bread, tortilla, cake, pancake, biscuit, crisp bread, cookie, piecrust, pizza, pasta, noodle or spaezle. Most preferably, the baked product is bread.

In addition to grains, seeds and liquid, the bread composition may contain also other suitable bread ingredients, including gluten, potato flour, sweetener, oil, emulsifiers, salt, and additives. Examples of suitable sweeteners include sucrose, high fructose corn syrup, brown sugar, honey, molasses, malt syrup or powder. The baked product may also comprise other conventional ingredients, e.g.: proteins, such as milk powder and soy; eggs (either whole eggs, egg yolks or egg whites); an oxidant such as ascorbic acid; and an amino acid such as L-cysteine.

All modifications of the method according to the invention, such as adding to the incubation mixture *Lactobacillus* expressing fructan degrading enzyme(s), preferably extracellular fructan hydrolase, in addition to the seed starter or a microbial starter are naturally within the scope of the invention.

While the following examples are illustrative of the principles of the present invention in one or more particular application, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also unrecited features. The features recited in depending claims are mutually freely combinable unless otherwise explicitly stated. Furthermore, it is to be understood that the use of "a" of "an", that is, a singular form, throughout this document does not exclude a plurality.

### Example 1. Spontaneous production of a seed starter.

A seed starter with the ability to quantitatively consume fructan can be produced from cut kernels without any pre-existing seed starter for instance in the following way. The cut kernels used in the example contain 0.2% of damaged starch.
1. 100 g of cut kernels of rye are soaked in 150 g of water and incubated at 45°C e.g. for 24h
2. 10 g of the mixture from step 1 is mixed with 100g of cut kernels of rye and 150 g of water and incubated at 45°C e.g. for 24h
3. 10 g of the mixture from step 2 is mixed with 100g of cut kernels of rye and 150 g of water and incubated at 45°C e.g. for 24h
4. 10 g of the mixture from step 3 is mixed with 100g of cut kernels of rye and 150 g of water and incubated at 45°C e.g. for 24h
5. 10 g of the mixture from step 4 is mixed with 100g of cut kernels of rye and 150 g of water and incubated at 45°C e.g. for 24h
6. 10 g of the mixture from step 5 is mixed with 100g of cut kernels of rye and 150 g of water and incubated at 45°C e.g. for 24h
7. 10 g of the mixture from step 6 is mixed with 100g of cut kernels of rye and 150 g of water and incubated at 45°C e.g. for 24h.

### Example 2.

A process for the production of low-fructan dried rye material is illustrated by the following flowchart:
1. Whole grain rye material ↓
2. Mixing the grain material with water and a seed starter prepared as in Example 1 containing specific lactic acid bacteria that are able to efficiently utilize fructan. ↓
3. Incubation at 40°C for 12 h ↓
4. Whole grain rye with low fructan content ↓
5. Drying ↓
6. Optional milling

### Example 3.

Sourdough-1 seed starter was prepared following the procedure from example 1 i.e. using cut rye grains and fermenting temperature of 45°C. The other three starters were rye flour fermented at 45°C (sourdough-4), coarse rye meal fermented at 30°C (sourdough-2), rye flour fermented at 30°C (sourdough-3). The decrease in fructan content of whole rye flour by using four different seed starters as a function of time is presented in Figure 1.

### Example 4.

A seed starter was prepared as described above. Bacterial colonies with different morphology (outlook) were isolated to pure cultures. These seed starter-derived pure culture isolates were used in the fermentation of cut rye kernels in water suspension. After 24-hour incubation the fructan content of samples were analyzed and compared to that of the starting raw material. Seed starter as such was used as a positive control. Isolate C appeared to be effective in fructan removal and was later on identified as *Lactobacillus crispatus.* Both Isolate C and the seed starter reduced the fructan content by more than 95% (see table below).

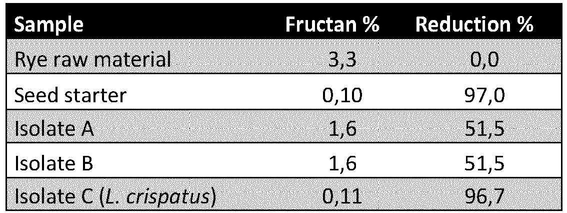

### Example 5.

Oligosaccharide profiles and fructan content of sourdough that is prepared using a seed starter prepared from rye grain material with damaged starch content of 0.2 (A) vs. similar sourdough system prepared using a seed starter prepared from rye grain material containing 2.7% of damaged starch (B).

In the example two different sourdoughs were compared with regard their fructan content profiles. Both sourdoughs were fermented for 16 hours at 40°C.

The fructan content was determined using the Megazyme Fructan kit by following the manufacturer's instructions. Fructan content of the raw materials i.e. whole grain cut rye kernels and whole grain rye flour were 4.3 % and 4.4 % (grain material basis), respectively. Fructan contents of the fermented sourdoughs A and B were 0.1 % and 3.7 % (grain material basis), respectively (Figure 2). This result demonstrates that fructans were quantitatively removed from sourdough A during the fermentation, while there was no change in the fructan content of sourdough B.

Samples of the two fermented sourdoughs were centrifuged and the liquid parts were analyzed with a HPAEC-PAD system that was set up for oligosaccharide analysis. In Figure 3, chromatogram peaks that are seen between 25 and 40 min represent mostly fructans and FOS. This result was verified by using fructanase treatment and consequent analysis. In Figure 3A, no major peaks between 25 and 40 min area exist. In Figure 3B, a number of peaks can be seen between 25 and 40 min. In conclusion, in system B fructans or FOS were present and thus were not quantitatively removed by starter microorganisms.

### Example 6.

Rye sourdough was prepared using fructan degrading seed starter prepared as in Example 1, cut rye kernels and water. The mixture was incubated at 40°C for 10 hours. **Left panel of** **Figure 4**: Mannitol started to form in the beginning of incubation. This is typical as the heterofermentative lactic bacteria reduce free fructose to mannitol. The mannitol concentration was at maximum after 5 h of incubation and thereafter it started to decline. This is because it was uptaken and consumed by starter microbes. After 10 h of incubation the system was substantially free from mannitol. **Right panel of** **Figure 4****:** Fructose content increased during the first two hours of incubation due to the fructan degradation. After 6 h of incubation, the system was substantially free from fructose.

### Example 7.

11 microbe colonies were isolated from a sourdough that was capable to reduce fructan levels by more than 90%. The microbes of these colonies were analyzed for their efficiency in removing fructan from grain material by using them as pure culture inoculants in laboratory fermentations. In each fermentation reaction, 20 g of cut grains of rye were mixed with 30 grams of tap water and 500 mg pure culture starter suspension containing 10⁹ cells of microbe isolate. After 16 hours fermentation at 37°C the fructan content of the mixtures was analyzed using a commercial kit (K-FRUC, Megazyme). The initial fructan content of the grain material was 5% (dry matter basis). After fermentation, only two of the isolates resulted in final fructan content below 0.5% (dm) (isolates 2 and 10) while the rest 9 isolates resulted in fructan content clearly above 1% (dm) (Fig. 5). The two effective pure cultures were sequenced using 16S rRNA gene identification and identified as strains belonging to *Lactobacillus ultunensis* species.

### Example 8.

The use of low-fructan grain material in baking mix-bread.
a) A seed starter with the ability to quantitatively consume fructan from grain material, as described in Example 4, can be used in bread making in various ways, and for instance, in the following manner. For preparation of sourdough for bread making, 1 kg of wholegrain rye flour is mixed with 1.5 kg of water and 90 g of the seed starter. The mixture is incubated for 8 hours at 42°C. After the incubation, more than 90% of the fructan has been removed.
b) For preparation of bread dough, 2 kg of flour ingredients (e.g. wheat, rye, oats, and barley) and 50 g of salt and 60 g of baker's yeast is added to the incubated sourdough of step a). The mix is molded to dough. The dough is allowed to rest for 2 hours at room temperature. The rested dough is molded into dough pieces with a desired format. The molded dough pieces are proofed at 37°C, 85% Rh for 1 hour. The proofed dough is baked at 200°C for 1 hour.

The final product is bread that has reduced fructan content compared to bread prepared identically using other sourdough than seed starter sourdough-1 from Example 3 or compared to bread with the same recipe but produced with a straight process (without the 1^{st} incubation stage).

### Example 9.

Influence of bread products with reduced amount of fructan on breath hydrogen levels and gastrointestinal symptoms.

Two different pumpernickel type rye breads with similar characteristics but differing in fructan contents were prepared following protocol described in previous Examples. The fructan content of Pumpernickel rye breads were:

| Bread | Fructan content, % (dry weight basis) |
|---|---|
| Pumpernickel bread 1 | 2.0 |
| Pumpernickel bread 2 | 0.5 |

Bread 1 represents reference bread with normal fructan content, while bread 2 had reduced fructan content. The breads were tested using postprandial test-design where the test subjects included 160 g of test bread to a standard breakfast. The amount of hydrogen in exhaled breath (ppm) was measured and various gastrointestinal symptoms (pain, bloating, flatulence, borborygmi, eructation, heartburn, diarrhea, nausea, defecation) were recorded. The results are presented in Figure 6.

The results of the study demonstrate that the amount of fructan ingested in bread directly reflects to the formation of gases during digestion as well as the gastrointestinal symptoms perceived by the test subjects.

### References

Andersson, R. Fransson, G., Tietjen, M. & Åman, P. (2009). Content and molecular-weight distribution of dietary fiber components in whole-grain rye flour and bread. Journal of Agricultural and Food Chemistry 57 (5), 2004-2008.
Fretzdorff, B. & Welge, N. (2003). Abbau von getreideeigenen Fructanen während der Herstellung von Roggen-Vollkornbrot. Getreide Mehl und Brot 57 (3) 147-151.
Rakha A., Åman, P. & Andersson, R. (2010). Characterisation of dietary fibre components in rye products. Food Chemistry 119 (3), 859-867.

### Patent literature

EP 2103219 A1
US 2011/0129572 A1
WO 2014/179843 A1

## Claims

1. A method for producing low-fructan grain material by means of lactic acid bacteria, said method comprising the steps of
- generating a seed starter from grain material by back slopping at least 6 times, wherein said grain material has a damaged starch content less than 0.5% by weight of said grain material, wherein the damaged starch content refers to a proportion of broken starch granules, to obtain the seed starter, which comprises lactic acid bacteria; and either
1)- mixing grain raw material with liquid and with said seed starter;
- incubating the obtained mixture at 20 to 50°C for 4 to 72 hours; and
- recovering the low-fructan grain material, which comprises 0 to 0.5% by weight of fructan; or
2)- isolating pure culture isolates from said seed starter, wherein the pure culture isolates comprise lactobacilli selected from *Lactobacillus ultunensis, L. crispatus, L. amylovorus, L. amylovorans, L. sobrius* and *L. acidophilus ;*
- mixing grain raw material with liquid and with said pure culture isolate(s);
- incubating the obtained mixture at 20 to 50°C for 4 to 72 hours; and
- recovering the low-fructan grain material, which comprises 0 to 0.5% by weight of fructan,
wherein said grain raw material is wheat, rye, or barley, or a mixture containing any of these.

2. The method according to claim 1, wherein the grain material used for generating the seed starter has a damaged starch content less than 0.25%, more preferably less than 0.1% by weight of said grain material.

3. The method according to claim 1, wherein the grain raw material in alternative 1) has a damaged starch content less than 1.0%, preferably less than 0.5%, more preferably less than 0.25%, even more preferably less than 0.1% by weight of said grain raw material.

4. The method according to claim 1, wherein the liquid is selected from the group consisting of water, carbonated water, milk, sour milk, cultured buttermilk, milk kefir, coconut kefir, water kefir, cultured yogurt, whey, lemon juice, apple cider vinegar, dilute acid and base and buffer.

5. The method according to claim 1, wherein the grain raw material in alternative 2) has a higher damaged starch content than 1.0% by weight of said grain raw material.

6. The method according to claim 1, wherein the pure culture isolates derived from said seed starter are used together with any other starter.

7. The method according to any one of claims 1-6, wherein said seed starter comprises lactobacilli, wherein said lactobacilli preferably comprise *Lactobacillus ultunensis, L. crispatus, L. amylovorus, L. amylovorans, L. sobrius* and/or *L. acidophilus.*

8. The method according to claim 1, wherein the mixture is incubated at 40 to 45°C for 6 to 24 hours.

9. The method according to any one of the preceding claims, wherein said seed starter or said pure culture isolates derived from said seed starter have the ability to consume more than 90% of fructan from the grain raw material.

10. The method according to claim 1, wherein said grain raw material contains at least 1 % (w/w) of fructan.

11. The method according to any one of the preceding claims, wherein the obtained low-fructan grain material is further subjected to wet-milling, extrusion, drying or dry-milling, pasteurizing, or adjusting its acidity level.

12. Low-fructan grain material obtainable by the method according to any one of the preceding claims, wherein the amount of each of fructo-oligosaccharides, fructose and mannitol is below 0.2% by weight and said low-fructan grain material comprises 0 to 0.5 % by weight of fructan, wherein said grain is wheat, rye, or barley.

13. Low-fructan grain material according to claim12, wherein said low-fructan grain material comprises 0 to 0.3 % by weight of fructan, preferably 0 to 0.1 % by weight of fructan.

14. Use of the low-fructan grain material according to claim 12 or 13 for making dough.

## Patentansprüche

1. Verfahren zur Produktion von Getreidematerial mit niedrigem Fructangehalt durch Milchsäurebakterien, wobei das Verfahren die Schritte umfasst zum
- Erzeugen eines Saatgutstarters aus Getreidematerial durch mindestens 6 mal Anfrischen, wobei das Getreidematerial einen geschädigten Stärkegehalt von weniger als 0,5 Gew.-% des Getreidematerials aufweist, wobei sich der geschädigte Stärkegehalt auf einen Anteil gebrochener Stärkegranula bezieht, um den Saatgutstarter zu erhalten, der Milchsäurebakterien umfasst; und entweder
1)- Mischen von Getreiderohmaterial mit Flüssigkeit und mit dem Saatgutstarter;
- Inkubieren des erhaltenen Gemisches 4 bis 72 Stunden bei 20 bis 50°C; und
- Gewinnen des Getreides mit niedrigem Fructangehalt, das 0 bis 0,5 Gew.-% Fructan umfasst; oder
2)- Isolieren reiner Kulturisolate von dem Saatgutstarter, wobei die reinen Kulturisolate Lactobacilli umfassen, ausgewählt aus *Lactobacillus ultunensis, L. crispatus, L. amylovorus, L, amylovorans, L. sobrius* und *L. acidophilus;*
- Mischen von Getreiderohmaterial mit Flüssigkeit und mit dem (den) reinen Kulturisolat(en);
- Inkubieren des erhaltenen Gemisches 4 bis 72 Stunden bei 20 bis 50°C; und
- Gewinnen des Getreidematerials mit niedrigem Fructangehalt, das 0 bis 0,5 Gew.-% Fructan umfasst,
wobei das Getreiderohmaterial Weizen, Roggen oder Gerste oder ein Gemisch ist, das eines davon enthält.

2. Verfahren nach Anspruch 1, wobei das Getreidematerial, das zum Erzeugen des Saatgutstarters verwendet wird, einen geschädigten Stärkegehalt von weniger als 0,25 Gew.-%, bevorzugter weniger als 0,1 Gew.-% des Getreidematerials aufweist.

3. Verfahren nach Anspruch 1, wobei das Getreiderohmaterial in Alternative 1) einen geschädigten Stärkegehalt von weniger als 1,0 Gew.-%, bevorzugt weniger als 0,5 Gew.-%, bevorzugter weniger als 0,25 Gew.-%, noch bevorzugter weniger als 0,1 Gew.-% des Getreiderohmaterials aufweist.

4. Verfahren nach Anspruch 1, wobei die Flüssigkeit ausgewählt ist aus der Gruppe bestehend aus Wasser, kohlensäurehaltigem Wasser, Milch, Sauermilch, kultivierter Buttermilch, Milchkefir, Kokosnusskefir, Wasserkefir, kultiviertem Joghurt, Molke, Zitronensaft, Apfelessig, verdünnter Säure und Base und Puffer.

5. Verfahren nach Anspruch 1, wobei das Getreiderohmaterial in Alternative 2) einen höheren geschädigten Stärkegehalt als 1,0 Gew.-% des Getreiderohmaterials aufweist.

6. Verfahren nach Anspruch 1, wobei die reinen Kulturisolate, die von dem Saatgutstarter abgeleitet sind, gemeinsam mit einem beliebigen anderen Starter verwendet werden.

7. Verfahren nach einem der Ansprüche 1-6, wobei der Saatgutstarter Lactobacilli umfasst, wobei die Lactobacilli vorzugsweise *Lactobacillus ultunensis, L. crispatus, L. amylovorus, L, amylovorans, L. sobrius* und/oder *L. acidophilus* umfassen.

8. Verfahren nach Anspruch 1, wobei das Gemisch 6 bis 24 Stunden bei 40 bis 45°C inkubiert wird.

9. Verfahren nach einem der vorstehenden Ansprüche, wobei der Saatgutstarter oder die reinen Kulturisolate, die von dem Saatgutstarter abgeleitet sind, die Fähigkeit aufweisen, mehr als 90% Fructan aus dem Getreiderohmaterial zu verbrauchen.

10. Verfahren nach Anspruch 1, wobei das Getreiderohmaterial mindestens 1 % (w/w) Fructan enthält.

11. Verfahren nach einem der vorstehenden Ansprüche, wobei das erhaltene Getreidematerial mit niedrigem Fructangehalt weiter Nassmahlen, Extrusion, Trocknen oder Trockenmahlen, Pasteurisieren oder Einstellung seines Säuregehalts unterzogen wird.

12. Getreidematerial mit niedrigem Fructangehalt, das durch das Verfahren nach einem der vorstehenden Ansprüche erhältlich ist, wobei die Menge jedes von Fructo-Oligosacchariden, Fructose und Mannitol unter 0,2 Gew.-% ist und das Getreidematerial mit niedrigem Fructangehalt 0 bis 0,5 Gew.-% Fructan umfasst, wobei das Getreide Weizen, Roggen oder Gerste ist.

13. Getreidematerial mit niedrigem Fructangehalt nach Anspruch 12, wobei das Getreidematerial mit niedrigem Fructangehalt 0 bis 0,3 Gew.-% Fructan, vorzugsweise 0 bis 0,1 Gew.-% Fructan umfasst.

14. Verwendung von Getreidematerial mit niedrigem Fructangehalt nach Anspruch 12 oder 13 zur Zubereitung von Teig.

## Revendications

1. Procédé pour produire un matériau de type graine à faible teneur en fructane au moyen de bactéries lactiques, ledit procédé comprenant les étapes consistant à
- générer un starter d'ensemencement à partir de matériau de type graine en repiquant au moins 6 fois, dans lequel ledit matériau à base de grains possède une teneur en amidon endommagé inférieure à 0,5 % en poids dudit matériau de type graine, dans lequel la teneur en amidon endommagé se rapporte à une proportion de granulés d'amidon cassés, pour obtenir le starter d'ensemencement, qui comprend des bactéries lactiques ; et soit
1)- mélanger du matériau brut de type graine avec un liquide et avec ledit starter d'ensemencement ;
- incuber le mélange obtenu à 20 à 50 °C pendant 4 à 72 heures ; et
- récupérer le matériau de type graine à faible teneur en fructane, qui comprend 0 à 0,5 % en poids de fructane ; soit
2)- isoler des isolats de culture purs dudit starter d'ensemencement, dans lequel lesdits isolats de culture purs comprennent des lactobacilles sélectionnés parmi *Lactobacillus ultunensis, L. crispatus, L. amylovorus, L. amylovorans, L. sobrius* et *L. acidophilus ;*
- mélanger du matériau brut de type graine avec un liquide et avec ledit ou lesdits isolats de culture purs ;
- incuber le mélange obtenu à 20 à 50 °C pendant 4 à 72 heures ; et
- récupérer le matériau de type graine à faible teneur en fructane, qui comprend 0 à 0,5 % en poids de fructane,
dans lequel ledit matériau brut de type graine est le blé, le seigle ou l'orge ou un mélange contenant l'un quelconque de ceux-ci.

2. Procédé selon la revendication 1, dans lequel le matériau de type graine utilisé pour générer le starter d'ensemencement possède une teneur en amidon endommagé inférieure à 0,25 %, plus préférablement inférieure à 0,1 % en poids dudit matériau de type graine.

3. Procédé selon la revendication 1, dans lequel le matériau brut de type graine dans l'alternative 1) possède une teneur en amidon endommagé inférieure à 1,0 %, de préférence inférieure à 0,5 %, plus préférablement inférieure à 0,25 %, encore plus préférablement inférieure à 0,1 % en poids dudit matériau brut de type graine.

4. Procédé selon la revendication 1, dans lequel le liquide est sélectionné à partir du groupe constitué par l'eau, l'eau gazéifiée, le lait, le lait fermenté, le babeurre de culture, le kéfir de lait, le kéfir de noix de coco, le kéfir d'eau, le yaourt de culture, le petit-lait, le jus de citron, le vinaigre de cidre, un acide et une base dilués et un tampon.

5. Procédé selon la revendication 1, dans lequel le matériau brut de type graine dans l'alternative 2) possède une teneur en amidon endommagé supérieure à 1,0 % en poids dudit matériau brut de type graine.

6. Procédé selon la revendication 1, dans lequel les isolats de culture purs dérivés dudit starter d'ensemencement sont utilisés conjointement avec un quelconque autre starter.

7. Procédé selon l'une quelconque des revendications 1 à 6, dans lequel ledit starter d'ensemencement comprend des lactobacilles, dans lequel lesdits lactobacilles comprennent de préférence *Lactobacillus ultunensis, L. crispatus, L. amylovorus, L. amylovorans, L. sobrius* et/ou *L. acidophilus.*

8. Procédé selon la revendication 1, dans lequel le mélange est incubé à 40 à 45 °C pendant 6 à 24 heures.

9. Procédé selon l'une quelconque des revendications précédentes, dans lequel ledit starter d'ensemencement ou lesdits isolats de culture purs dérivés dudit starter d'ensemencement possèdent l'aptitude à consommer plus de 90 % du fructane issu du matériau brut de type graine.

10. Procédé selon la revendication 1, dans lequel ledit matériau brut de type graine contient au moins 1 % (p/p) de fructane.

11. Procédé selon l'une quelconque des revendications précédentes, dans lequel le matériau de type graine à faible teneur en fructane est en outre soumis à un broyage humide, une extrusion, un séchage ou un broyage à sec, une pasteurisation ou un ajustement de son niveau d'acidité.

12. Matériau de type graine à faible teneur en fructane pouvant être obtenu par le procédé selon l'une quelconque des revendications précédentes, dans lequel la quantité de chacun des fructo-oligosaccharides, du fructose et du mannitol est inférieure à 0,2 % en poids et ledit matériau de type graine à faible teneur en fructane comprend 0 à 0,5 % en poids de fructane, dans lequel ladite graine est le blé, le seigle ou l'orge.

13. Matériau de type graine à faible teneur en fructane selon la revendication 12, dans lequel ledit matériau de type graine à faible teneur en fructane comprend 0 à 0,3 % en poids de fructane, de préférence 0 à 0,1 % en poids de fructane.

14. Utilisation du matériau de type graine à faible teneur en fructane selon la revendication 12 ou 13 pour préparer une pâte.
